# EUROPEAN PATENT APPLICATION

(11) **EP 3 770 915 A1**
(43) Date of publication of application: **27.01.2021**
(21) Application number: 20181795.4
(22) Date of filing: 23.06.2020
(51) Int. Cl.: G16H 40/20

(54) **METHODS AND SYSTEMS FOR IMPROVING INFECTION CONTROL IN A FACILITY**

(30) Priority: 25.07.2019 US 201916522326
(71) Applicant: Honeywell International Inc., Morris Plains, NJ 07950 (US)
(72) Inventor: WELLIG, Armin, Morris Plains, NJ New Jersey 07950 (US)
(74) Representative: Houghton, Mark Phillip

(57) **Abstract**

Methods and systems for monitoring procedural compliance of staff in a facility. One or more defined procedure to be performed by staff in a room of the facility are stored in a memory. The defined procedure(s) may include one or more conditions that must be met for the procedure to be considered as performed correctly. The one or more conditions may include visiting a defined procedure location in the room. The location of the person in the room may be identified by triangulating one or more signals emitted by a device carried by the person and/or transmitting one or more signals into the room and receiving one or more corresponding reflected signals reflected off the person in the room. The method and/or system may determine whether the one or more conditions of the defined procedure have been met, and if not, issue an alert.

## Description

### Technical Field

The disclosure generally relates to procedural compliance of staff in a medical treatment facility, and more particularly to systems and methods for monitoring and/or correcting procedural compliance of staff in a medical treatment facility to reduce the risk of Hospital Acquired Infections (HAI) for both patients and staff.

### Background

Hospital Acquired Infections (HAI) and/or Surgical Site Infections (SSI) are infections caused by virus, bacteria and other environmental factors acquired within hospitals or other medical treatment facilities. It is estimated that HAI and SSI infections cost the healthcare industry nearly $40 billion annually. HAI and SSI infections can be transmitted in multiple ways, including, but not limited to, surface contamination, airborne particulates and aspiration. HAI and SSI infections can be reduced by following strict procedures, including procedures regarding decontamination practices, hand hygiene/antisepsis procedures, and other procedures. However, rather low compliance to such procedures among staff have been reported. What would be desirable is a method and system to help improve compliance with such procedures and to provide a notification when such procedures are not followed.

### Summary

This disclosure relates to procedural compliance of staff in a medical treatment facility, and more particularly to systems and methods for monitoring and/or correcting procedural compliance of staff in a medical treatment facility to reduce the risk of Hospital Acquired Infections (HAI) for both patients and staff.

In a first example, a method for monitoring procedural compliance of staff in a facility includes storing a defined procedure that is to be performed by a person in a room of the facility. The defined procedure includes one or more conditions, wherein the one or more conditions includes visiting a defined procedure location in the room. The method may further include identifying a location of a person in a room of the facility over time, including triangulating off one or more signals emitted by a device carried by the person and/or transmitting one or more signals into the room and receiving one or more corresponding reflected signals reflected off the person in the room. The method may further include determining whether the one or more conditions of the defined procedure have been met, including determining whether the person visited the defined procedure location in the room.

In another example, a system for monitoring procedural compliance of staff in a facility may include a memory for storing a defined procedure that is to be performed by a person in a room of the facility, wherein the defined procedure includes one or more conditions and the one or more conditions includes visiting a defined procedure location in the room. The system may further include a location identifier for identifying a location of a person in a room of the facility over time, wherein the location identifier is free from imaging pixels that together can be used to form a visually perceptible image of the room, and a controller operatively coupled to the memory and the location identifier, the controller configured to determining whether the one or more conditions of the defined procedure have been met, including determining whether the person visited the defined procedure location in the room.

In another example, a method for monitoring procedural compliance of staff in a facility may include determining when a person is at a defined procedure location in a room of the facility, determining a length of time the person is in the defined procedure location, and delivering an alert to the person when the person has failed to hover at the defined procedure location for at least a threshold period of time (which may also be referred to as dwell time).

The preceding summary is provided to facilitate an understanding of some of the features of the present disclosure and is not intended to be a full description. A full appreciation of the disclosure can be gained by taking the entire specification, claims, drawings, and abstract as a whole.

### Brief Description of the Drawings

The disclosure may be more completely understood in consideration of the following detailed description of various embodiments in connection with the accompanying drawings, in which:
Figure 1 is a schematic block diagram of an illustrative system for monitoring procedural compliance of staff in a facility;
Figure 2 is a flow chart of an illustrative method for monitoring procedural compliance of staff in a facility;
Figure 3 is an illustrative chart showing illustrative defined procedures; and
Figure 4 is a schematic view of a room in a facility including an illustrative system for monitoring procedural compliance of staff in a facility.

While the disclosure is amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the spirit and scope of the disclosure.

### Description

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

Hospital Acquired Infections (HAI) and/or Surgical Site Infections (SSI) are infections caused by virus, bacteria and other environmental factors acquired within hospitals or other medical treatment facilities. HAI and SSI infections can be transmitted in multiple ways, including, but not limited to, surface contamination, airborne particulates and aspiration. HAI and SSI infections can be reduced by following strict procedures, including procedures regarding decontamination practices, hand hygiene/antisepsis procedures, and other procedures. This may include, but not limited to, operating room personnel, physicians, nursing staff, custodial staff, etc. However, rather low compliance to such procedures among staff have been reported.

One example of a procedure where compliance is important is in an operating room (OR). In the case of an OR, all sterile team members should perform a hand and arm scrub before entering the surgical suite. The basic principle of the scrub is to wash the hands thoroughly, and then to wash from a clean area (the hand) to a less clean area (the arm). A systematic approach to the scrub is an efficient way to ensure proper technique. There are typically two methods of scrub procedure. One is a numbered stroke method, in which a certain number of brush strokes are designated for each finger, palm, back of hand, and arm. An alternative method is a timed scrub, and each scrub should last from three to five minutes, depending on facility protocol.

Another example of a procedure where compliance is important is cleaning and/or sterilizing of rooms. For example, critical cleaning procedures may specify one or both of an order or route of cleaning (e.g., clean A before B) and/or a minimum dwell time (e.g., a length of time that the person doing the cleaning should remain in a particular area for the cleaning or disinfection to be properly done. Since many preventative measures (e.g., hand hygiene, disinfection, etc.) include human activity performed by medical and/or cleaning staff, it is desirable to implement technologies that can track and enforce compliance with these and other preventative measures.

This disclosure generally relates to method and systems for monitoring procedural compliance of staff in a facility to cleanliness practices (e.g., handwashing, room cleaning, etc.) and for providing real time notification to the staff when a procedure is performed incorrectly or not at all while maintaining patient and/or staff privacy. While the system is described with respect to a medical facility, it is contemplated that the system may be used in other environments to ensure procedural compliance, such as, but not limited to, senior care facilities, nursing homes, restaurants, hotels, office buildings, etc.

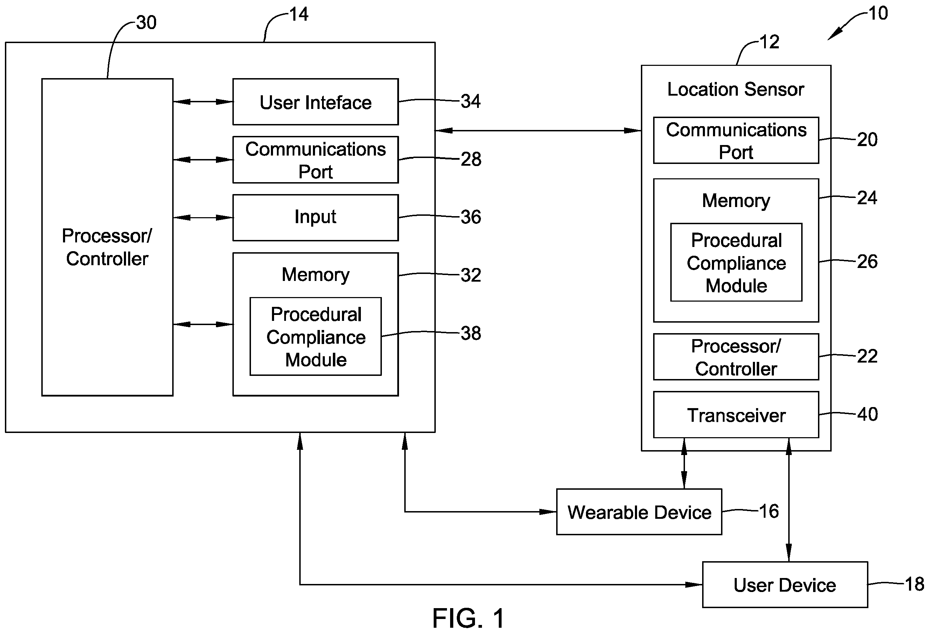

Figure 1 is a schematic block diagram of an illustrative system 10 for monitoring procedural compliance of staff in a hospital or other clinical setting. The system 10 may allow for precise people tracking without the use of cameras. For example, the system 10 may be configured to identify a location of a person in a room and determine if they have performed a task based on the location of the person and a length of time the person remained in that location (which may also be referred to as a dwell time). Generally, the system 10 may include a location identifier or sensor 12 and a computing device 14. In some cases, the information processing may be performed at the location sensor 12 and a separate computing device 14 may not be required. The system 10 may optionally include a wearable device 16 (e.g., a watch, a bracelet, a fitness tracker, an identification badge, etc.) and/or a user device 18 (e.g., a cell phone, a tablet computer, etc.) carried by the person entering the room.

In some cases, the location sensor 12 may be configured to transmit or emit one or more low power radio signals (e.g., an indoor wave radar system). For example, the location sensor 12 may include a transceiver 40 that is configured to both transmit signals into a space and receive signals reflected off of objects within the space. In another example, the location sensor 12 may be part of a number of distributed beacons that can be used to identify the location of a wearable device 16 and/or user device 18 of a person by triangulating among signals received at the beacons from the wearable device 16 and/or the user device 18.

In some cases, the location sensor 12 may be configured to transmit one or more signals in the millimeter band. For example, the location sensor 12 may be configured to transmit signals in the range of 30 gigahertz (GHz) to about 300 GHz. In some instances, the location sensor 12 may be configured to transmit signals of about 60 GHz. In some cases, the location sensor 12 may be configured to transmit one or more signals outside of the millimeter band, if so desired. For example, the location sensor may be configured to transmit a micro-wave signal (e.g., in the range of about 300 megahertz (MHz) to about 30 GHz). It is further contemplated that the location sensor 12 may be based on LIDAR, infrared, or any other non-optical wavelength, etc.

The location sensor 12 may identify a position of an object (e.g. person) by measuring the length of time it takes for the transmitted radio signal to reflect off of an object and return to a location sensor 12, as will be described in more detail herein. In some cases, the location sensor 12 may be positioned centrally in a room and configured to transmit one or more signals throughout an entire room (e.g., 360°). In some cases, the location sensor 12 may be mounted at or near the ceiling. It is contemplated that the system 10 may include more than one location sensor 12, depending on the size and/or shape of the room.

The location sensor 12 may include a communications port 20 for operatively coupling to the computing device 14, the wearable device 16, and/or the user device 18. It is contemplated that the communications port 20 may be wired and/or wireless. When the communications port 20 is wireless, the communications port 20 may include a wireless transceiver, and the computing device 14, the wearable device 16, and/or the user device 18 may include a compatible wireless transceiver. It is contemplated that the wireless transceivers may communicate using a standard and/or a proprietary communication protocol. Suitable standard wireless protocols may include, for example, cellular communication, ZigBee, Bluetooth, WiFi, IrDA, dedicated short range communication (DSRC), EnOcean, or any other suitable wireless protocols, as desired.

The location sensor 12 may include one or more controllers or processors 22 that execute instructions stored in the system memory 24. In some cases, the controller 22 may include a programmable microprocessor. Such a programmable microprocessor may allow a user to modify the control logic of the location sensor 12 even after it is installed in the field (e.g., firmware update, application update). The system memory 24 of the location sensor 12 can include computer system readable media in the form of volatile memory, such as random access memory (RAM) and/or cache memory. The location sensor 12 may further include other removable/non-removable, volatile/non-volatile computer system storage media. By way of example only, the storage system can be provided for reading from and writing to a non-removable, non-volatile magnetic media (not shown and typically called a "hard drive" or flash memory). The system controller 22 and/or memory 24 may include at least one program/utility having a set of program modules that are configured to receive an input from or transmit an output to a remote computing device 14, a wearable device 16, and/or a user device 18.

In one example, the program/utility may be stored in the system memory 24 and may include one or more application program modules (e.g., software), such as a procedural compliance module 26. The procedural compliance module 26 may include one or more defined procedures that should be performed in a particular room of a facility in order to reduce the risk of infection. Each defined procedure may include one more conditions or actions that should be performed in order for the procedure to have been considered as performed correctly or to a standard, as will be described in more detail herein. It is contemplated that the defined procedure may be different for different types of rooms and/or a category type of the person entering the room. For example, the defined procedures for an OR may be different for the defined procedures for a patient room. Also, the defined procedures for a nurse may be different for the defined procedures for a member of the custodial staff. In some cases, the procedural compliance module 26 may be a part of the location sensor 12 or may be executed on the remote computing device 14, as will be described in more detail herein.

As described herein, the computing device 14 may be operatively coupled to the location sensor 12. The computing device 14 may be a system specific controller, a desktop computer, a laptop computer, a tablet, a cloud based computing device, etc. When the location sensor 12 is equipped with a processing device 22 and memory 24, the computing device 14 may not be required for the function of the system 10. When the computing device is provided, the computing device 14 may be located in a same room as the location sensor 12, in a different room but a same building as the location sensor 12, or off-site (e.g., geographically different location) from the location sensor 12, as desired. The computing device 14 may include a communications port 28 for communicating with the location sensor 12 (or other devices, such as but not limited to, cell phones, wearable devices, etc.), the wearable device 16, and/or the user device 18. In some cases, the computing device 14 may include more than one communications port 28 for communicating over more than one network (e.g., wireless LAN, wired LAN, the Internet, short range wireless communications, etc.).

The computing device 14 may further include a processor 30 (e.g. microprocessor, microcontroller, etc.) and a memory 32. The computing device 14 may also include a user interface 34. The user interface 34 may be a display or other means for allowing a user to interact with the computing device 14. The user interface 34 may be part of a personal computer, tablet computer, smart phone, laptop computer, or may include a standalone display. In some instances, the computing device 14 may include a user input 36 for receiving a user input from a user. For example, the user input may include a keyboard, mouse, actuatable buttons, or a touchscreen display. These are just examples.

The memory 32 may be used to store any desired information, such as the aforementioned procedural compliance module. The memory 32 may be any suitable type of storage device including, but not limited to, RAM, ROM, EPROM, flash memory, a hard drive, and/or the like. In some cases, the processor 30 may store information within the memory 32, and may subsequently retrieve the stored information from the memory 32. In some cases, the memory 32 may store one or more application program modules (e.g., software), such as a procedural compliance module 38. The procedural compliance module 38 may be the same as or different from the procedural compliance module 26 associated with the location sensor 12. The procedural compliance module 38 may include one or more defined procedures that should be performed in a particular room of a facility in order to reduce the risk of infection. Each defined procedure may include one more conditions or actions that should be met in order for the procedure to have been considered as performed correctly or to a standard, as will be described in more detail herein. It is contemplated that the defined procedure may be different for different types of rooms and/or a category type of the person entering the room (e.g., custodial staff, medical staff, etc.). In some cases, only one of the location sensor 12 or the computing device 14 is provided with a procedural compliance module 26, 38. In other cases, both the location sensor 12 and the computing device 14 are provided with a procedural compliance module 26, 38.

The wearable device 16 may be device that is worn by or carried by a person (e.g., staff member). In some cases, the wearable device 16 may allow the system 10 to identify the staff member or a staff member type (e.g., physician, nurse, custodian, food service, etc.) when they enter a room. The identity of the staff member may then be used to determine which procedure the staff is expected to comply with when entering a particular room. In some embodiments, the wearable device 16 may be used to identify the location of the person in the room. For example, the location of the person may be identified by triangulating off one or more signals emitted by the wearable device 16, as will be described in more detail herein.

In one example, the wearable device 16 may be a network or internet enabled bracelet or watch. It is contemplated that in addition to providing information regarding the staff, a bracelet may be equipped with gesture tracking technology. When so provided and worn on a wrist, a wearable device 16 may be used to provide additional information which may be used to determine if certain procedures are being followed (e.g. hand sanitizing, hand washing, cleaning designated surfaces, etc.), as will be described in more detail herein. In other examples, the wearable device 16 may be an employee badge with a radiofrequency (RF) tag. It is contemplated that other wearable devices 16 may also be used, including, but not limited to, rings, necklaces, cell phones, etc. In some cases, the wearable device 16 may be include a wireless transceiver to wirelessly communicate with the location sensor 12, the computing device 14 and/or the user device 18. When so provided, it is contemplated that the wireless transceivers may communicate using a standard and/or a proprietary communication protocol. Suitable standard wireless protocols may include, for example, cellular communication, ZigBee, Bluetooth, WiFi, IrDA, dedicated short range communication (DSRC), EnOcean, or any other suitable wireless protocols, as desired. In some embodiments, the system 10 may not include a wearable device 16.

The user device 18 may be a device carried by a staff member as they are performing their duties. The user device 18 may include, but is not limited to, mobile devices including smart phones, tablet computers, laptop computers, wireless network-enabled key fobs, e-readers, and/or the like. The user device 18 may be configured to receive messages from the location sensor 12 and/or the computing device 14 regarding a procedure that is expected to be performed in a particular room. In some cases, the messages may be informing the staff of the procedural requirements. In other cases, the messages may be informing the staff they have failed to perform a procedure or performed it incorrectly, as will be described in more detail herein. In some embodiments, the system 10 may not include a user device 18.

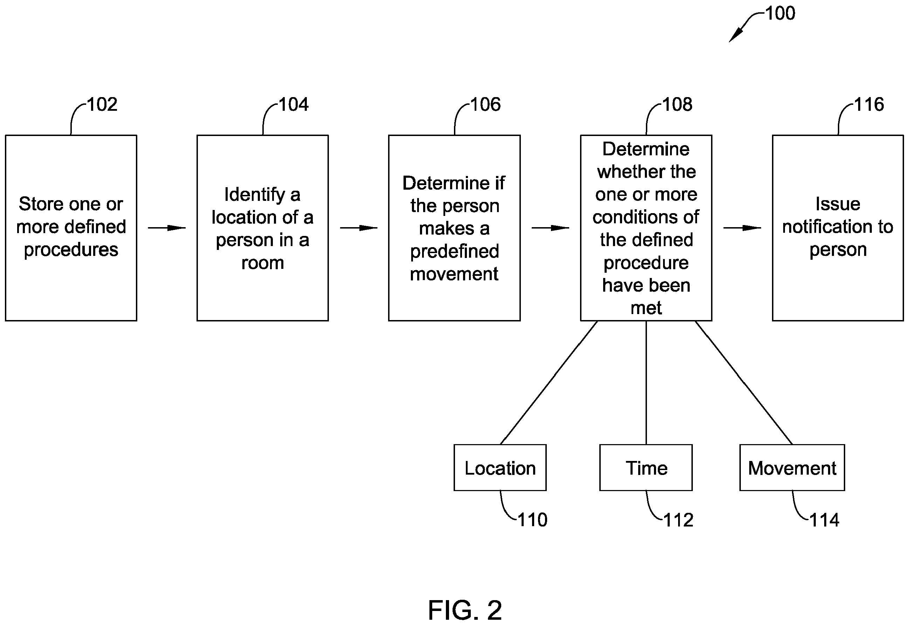

Figure 2 is a flow chart of an illustrative method 100 for using the system 10 of Figure 1 to monitor procedural compliance of staff in a facility. To begin, one or more defined procedures that are to be performed by a person in one or more rooms of the facility may be defined and stored in the memory 24, 32 of the location sensor 12 and/or computing device 14, as shown at block 102. It is contemplated that when the one or more defined procedures are stored in the memory 24 of the location sensor 12, the one or more defined procedures may be specific to the particular room in which the location sensor 12 is located. In other cases, all of the defined procedures, regardless of the room type to which they apply, may be stored in the same database (e.g., in the same memory). Each defined procedure may include one or more conditions that must be met for the defined procedure to be considered correctly completed. In some cases, at least one of the conditions may include the person visiting a defined procedure location in the room (e.g. hand sanitizer station or sink). In other words, each defined procedure has a location where the defined procedure is expected to occur.

For example, when entering a patient room, a physician or nurse may be expected to wash their hands at a sink. Thus, when the physician or nurse enters the patient room, the defined procedure location would correspond to a location adjacent the sink. In another example, when entering a patient room, a physician or nurse may be expected to apply hand sanitizer at hand sanitizing station. Thus, when the physician or nurse enters the patient room, the defined procedure location would correspond to a location adjacent the hand sanitizing station. In yet another example, when entering a room, a custodian may be expected to clean one or more surfaces, such as, but not limited to a counter and/or empty a trash receptacle. Thus, when the custodian enters the patient room, the defined procedure location would correspond to the counter surface that should be cleaned (sometimes on a scheduled basis) and/or the location of the trash receptacle. The defined procedure locations may be defined or programmed during commissioning of the location sensor 12 and/or computing device 14. In some cases, the defined procedure location may be updated or changed after commissioning.

In some embodiments, the defined procedure may include more than one defined procedure location. For example, when determining if a room has been properly cleaned, there may be more than one defined procedure location. When the one or more conditions of the defined procedure includes more than one defined procedure location, the person must visit all of the defined procedure locations in the room. It is further contemplated that the one or more conditions may include visiting the defined procedure location according to a predefined schedule. For example, a nurse making rounds may need to visit each patient room on consistent basis, or custodial staff must clean designated surface on a daily basis. If these do not occur, the defined procedure may be considered to be violated.

In some cases, at least one of the conditions of the defined procedure may include hovering (e.g., remaining, pausing waiting, etc.) at the defined procedure location for at least a predetermined procedure time (e.g., a dwell time). For example, the person may be required to scrub their hands for a predetermined minimum length of time. Monitoring a length of time a person is at the defined procedure location may also help prevent false positives when a person merely passes through the defined procedure location. For example, a hand sanitizing station may be positioned just inside of a doorway. A person may pass the hand sanitizing station, and thus be at defined procedure location without actually using the hand sanitizer. Associating a length of time with the defined procedure location may help reduce false positives. Other temporal conditions may be used as desired. For example, in the case of a patient room having a patient bed, the person may be required to visit the defined procedure location (e.g., for hand hygiene) prior to visiting the location of the patient's bed. Thus, the person would enter the room, visit the defined procedure location before visiting the patient's bed for the one or more conditions to have been met.

In some embodiments, at least one of the conditions of the defined procedure may be a predefined movement of the person at the defined procedure location. For example, when washing hands, a person is expected to make a hand washing movement. This movement may be captured by, for example, a wearable device 16 (such as, but not limited to, a watch or bracelet) and transmitted to the location sensor 12 or the computing device 14. In some embodiments, the movement may be captured by the location sensor 12 itself. For example, a location sensor 12 operating in the 60 GHz frequency range or more with a very large bandwidth may provide centimeter or even millimeter spatial resolution. In some cases, it is contemplated that the location sensor 12 may obtain location in the range of about eight to ten frames per second. Other predefined movements may include, but are not limited to, a hand sanitizer pump movement, a wiping or scrubbing movement (for cleaning), a lifting movement (e.g., emptying trash receptacles), etc. These illustrative movements are not meant to be inclusive of all predefined movements, but rather merely representative of some motions or movements that can be used to verify procedural compliance.

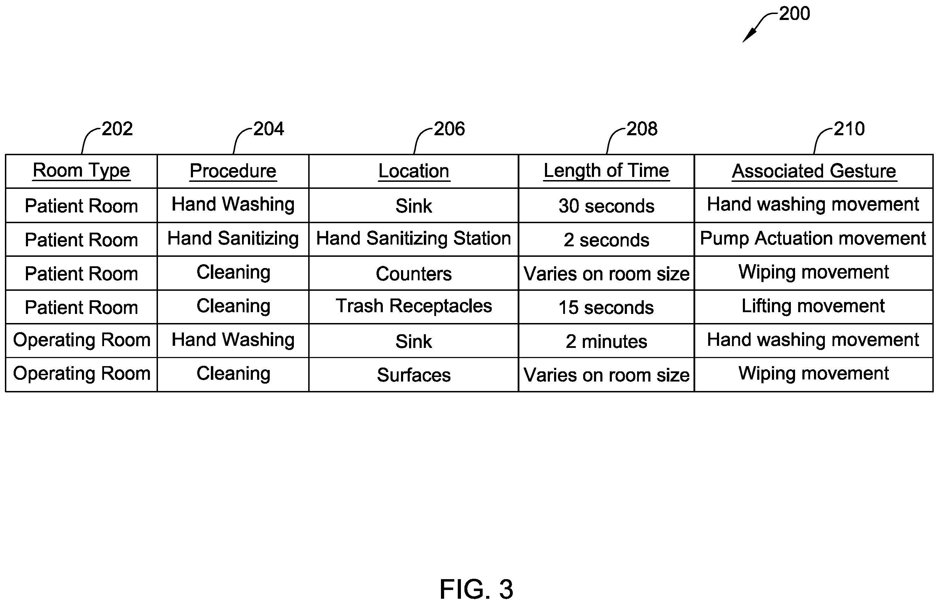

Figure 3 is table 200 showing some illustrative defined procedures 204 that may occur in a particular room type 202, the defined procedure location 206, a length of time 208 the person should remain in the defined procedure location 206, and a gesture or movement 210 that accompanies the defined procedure. These illustrative defined procedures 204 are not meant to be inclusive of all possible defined procedures, but rather merely representative of defined procedures that can be verified. While not explicitly shown, in some cases, one or more conditions of the defined procedure may be dependent, at least in part, on a sensed condition of an environmental sensor. Some illustrative environmental sensors may include, but are not limited to, VOC sensors (e.g., to determine if a specific detergent is used), temperature sensors, humidity sensors, luminance sensors (e.g., occupancy sensors), microphones (e.g., to listen for running water), etc.

Returning to Figure 2, once the defined procedures have been stored, the system 10 may be used to monitor for procedural compliance. The system 10 may be configured to identity a location of the person entering the room, as shown at block 104. It is contemplated that the location can be determined in a number of different ways. In a first example, the location sensor 12 may transmit one or more signals into the room and receive one or more corresponding reflected signals reflected off the person in the room (e.g. indoor radar). In some cases, the location sensor 12 may emit a signal in the millimeter wave or micro-wave range which functions as a miniaturized radar system providing a 3-dimensional image created by processing the reflections of the radiated signals. Said differently, the location of the person in the room may be identified by an in-room wave radar system (e.g., location sensor 12) that transmits one or more micro-wave or millimeter wave signals into the room. For example, the locations sensor 12 may provide the x, y and z coordinates of both stationary objects (furniture, etc.) and moving objects (e.g., people, carts, etc.). In some cases, the in-room wave radar system may include a plurality of distributed antenna for receiving a plurality of reflected signals (e.g., point cloud) reflected off the person in the room. In some embodiments, the distributed antenna may be arranged along all four sides (for a rectangular sensor device) of the radar device (e.g., location sensor 12) to receive reflected signals from an entirety of the room. For a non-rectangular device 12, the distributed antenna may be arranged uniformly about a perimeter of the device. As the millimeter wave (or micro-wave) signals do not include an optical component, personal privacy and data privacy is respected. It is contemplated that when the location sensor 12 is installed, a commissioning process is performed to teach the system 10 the stationary objects in the room as well as to identify the defined procedure locations. In another example, the location of a person in a room of a facility can be identified by triangulating off one or more signals emitted by a device carried by the person. For example, signals emitted by the wearable device 16 may be triangulated or signals emitted by the user device 18 may be triangulated.

The system 10 may be further configured to determine if the person is making or performing a predefined movement, as shown at block 106. It is contemplated that the system 10 may identify the location of the person and determine if the person is making a predefined movement substantially simultaneously. In other embodiments, the system 10 may first identify the location of the person and then "watch" for the predefined movement while the person is in the defined procedure location. In some instances, the identity of the person or a job category of the identified person entering them room may be transmitted or relayed to the system 10 via a wearable device 16 and/or the user device 18. This may allow the system 10 to determine what defined procedure should be performed and what conditions should be met in order for the person to have complied with the procedure.

The system 10 may be configured to determine whether or not the one or more conditions of the defined procedure have been met, as shown at block 108. The system 10 may be configured to compare the location 110 of the person, a length of time 112 the person has remained in the defined procedure location and/or any movements 114 performed while in the defined procedure location to the one or more conditions of the defined procedures. If the person has met the one or more conditions of the defined procedure (e.g., location 110, time 112, and/or movement 114), the system 10 may take no further action. If the person has not met the one or more conditions of the defined procedure, the system 10 may provide an alert, as shown at block 116. It is contemplated that the alert may be provided in real time so that the person has a chance to correct their mistake. In some cases, the alert may be provided directly to the user device 18 and/or the wearable device 16 of the person in the room. An illustrative alert may remind the user to perform the defined procedure (e.g., hand hygiene, custodial tasks, etc.). In other cases, an alert may be provided to a central location, such as, but not limited to, a nurses' station, supervisor, management, etc. In some cases, the room itself may have an alerting device that can annunciate the alert to the person in the room.

In an illustrative example, an alert may be provided when a nurse or physician enters a patient room but fails to pause or stop at the hand sanitizing station or at the sink. The alert may remind the user of the hand hygiene procedure. It is contemplated that the alert may be a plain text alert (e.g., please wash your hands or please return to the hand sanitizer, etc.), a visual alert (e.g., an illuminated light), an audible alert (e.g., a beep or series of beeps), a haptic alert (e.g., a vibration of the wearable device 16 or the user device 18), etc.

In another example, the movement of a person in a room can be tracked and a heat map of the location of the person recorded. For example, as a custodian moves around a room, locations in which the custodian has spent the most time will appear as "hotter" on the heat map. This heat map may be analyzed and scored to determine if the custodian has met the procedural guidelines for cleaning the particular room. In some cases, critical cleaning process steps may be specifically tracked. If the score is below a predetermined threshold, an alert may be provided to the custodian and/or other staff.

In yet another example, the movements of a patient in the room may be tracked. For example, if the patient is started on a new therapy it may be desirable to be aware of side effects that the patient is experiencing. Thus, if a patient begins using a restroom more or becomes restless (these are just some examples) or if the patient remains at an unexpected location for a period of time (e.g., has fallen on the floor), the medical staff can be alerted.

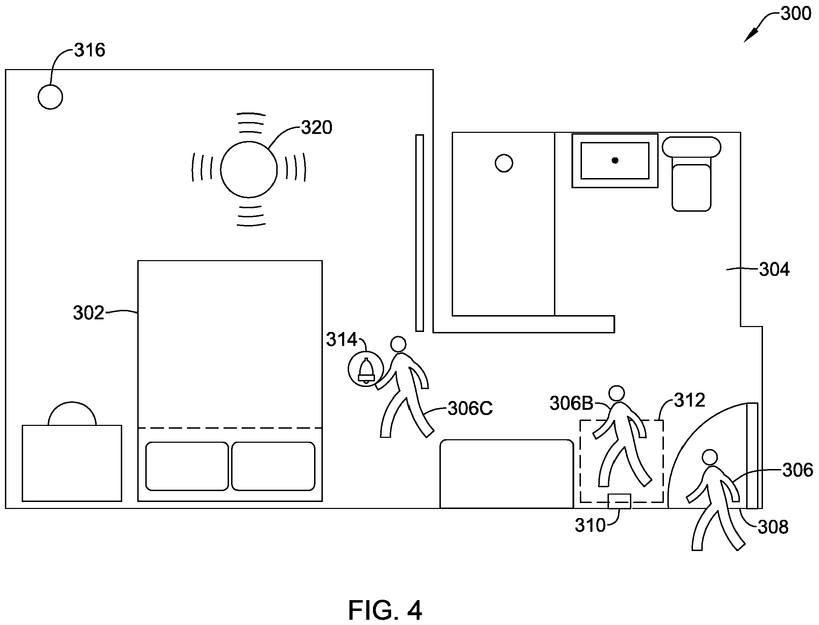

Figure 4 is a schematic view of an illustrative patient room 300 of a hospital or other clinical setting including a system 10 for monitoring procedural compliance of staff in a patient of a hospital or other clinical setting. The patient room 300 may include a patient bed 302 and other fixed location furniture. The patient room 300 may also include bathroom facilities 304. The patient room 300 is provided with a location identifier 320. The location identifier may be a location sensor similar in form and function to the location sensor 12 described herein or may be a processor configured to triangulate the location off one or more signals emitted by a device carried by the person 306. As noted above, the system 10 may include a memory for storing one or more defined procedures that are to be performed by a person 306 in the 300 room of the facility. The memory 24, 32 may be a part of either or both of the location sensor 12 (or location identifier 320) and/or the computing device 14. Each of the defined procedures may include one or more conditions that indicate the procedure has been performed correctly. As described herein each of the defined procedures may require the person 306 to visit a defined procedure location 312 in the room.

In the illustrative example of Figure 4, the person 306 entering the room 300 through a doorway 308 is medical staff. It is contemplated that the person may be identified via a wearable device such as an employee badge, smart watch, or internet connected bracelet or by a user device such as a smart phone or tablet. The person 306 may be expected to follow a hand hygiene procedure when entering the room 300. A hand sanitizing station 310 is positioned just inside the doorway 308 with a defined procedure location 312 adjacent to the hand sanitizing station 310. The location identifier 320 may identify a location of the person 306 in the room 300 over time (e.g., as the person moves through the room 300). As described above, both an indoor radar system and triangulation of signals from a device of the person 306 are free from imaging pixels that together can be used to form a visually perceptible image of the room. Thus, both patient and staff data and personal privacy are respected. A controller (e.g., which may be a part of the location identifier 320 or separate computing device in communication with the location identifier 320) may be operatively coupled to the memory and the location identifier 320 (e.g., the location data). The controller may be configured to determine whether the one or more conditions of the defined procedure have been met.

In the illustrative example of Figure 4, the controller may be configured to determine if the person 306b has visited the defined procedure location 312 (e.g., adjacent to the hand sanitizing station 310) in the room 300. The controller may be further configured to determine if the person 306b has hovered or paused at the defined procedure location 312 to use the hand sanitizing station 310. For example, the location identifier 320 may provide an updated location to the controller at a rate of greater than 5 times per second such that a slight pause to actuate the hand sanitizer pump is detectable. The controller may also be configured to determine if the person 306b has performed a movement associated with using the hand sanitizing station 310. For example, the controller may be configured to identify an actuation of the pump of the hand sanitizer or the movement of the person's 306b hands as they apply the hand sanitizer.

If the person 306c enters the room and bypasses the hand sanitizing station 310, an alert 314 may be sent to the user (e.g., via a wearable device or user device as described herein) reminding the user to return to the hand sanitizing station 310. In other words if the person 306c has failed to hover at the defined procedure location 312 for at least a threshold period of time (e.g., just passes by the defined procedure location 312), an alert 314 may be delivered to the person 306c (or supervisor, etc.).

The patient room 300 may further include one or more environmental sensors 316. Some illustrative environmental sensors 316 may include, but are not limited to, VOC sensors (e.g., to determine if a specific detergent is used), temperature sensors, humidity sensors, luminance sensors (e.g., occupancy sensors), microphones (e.g., to identify running water), etc. The environmental sensors 316 may provide additional data to determine when the defined procedure should be followed. For example, when a patient room 300 is unoccupied it may not be necessary for medical staff to follow the hand hygiene procedure. This is just one example of how additional contextual data can be used.

Figure 4 is one illustrative example and is not intended to be limiting. As set forth above, the defined procedures may be unique to one or more of a room type of the room and/or a classification type of the person in the room. Further, the patient room 300 (or other room) may be provided with more than one location identifier 320 as desired. For example, while the radar waves (e.g., of an indoor radar system) may pass through walls, additional sensors may improve accuracy. It is further contemplated that location identifier 320 can be zoned or set up to monitor a specific area during commissioning.

Those skilled in the art will recognize that the present disclosure may be manifested in a variety of forms other than the specific embodiments described and contemplated herein. Accordingly, departure in form and detail may be made without departing from the scope and spirit of the present disclosure as described in the appended claims.

## Claims

1. A method for monitoring procedural compliance of staff in a facility, the method comprising:
storing a defined procedure that is to be performed by a person in a room of the facility, wherein the defined procedure includes one or more conditions, wherein the one or more conditions includes visiting a defined procedure location in the room;
identifying a location of a person in a room of the facility over time, including:
triangulating off one or more signals emitted by a device carried by the person; and/or
transmitting one or more signals into the room and receiving one or more corresponding reflected signals reflected off the person in the room; and
determining whether the one or more conditions of the defined procedure have been met, including determining whether the person visited the defined procedure location in the room.

2. The method of claim 1, wherein the location of the person in the room is identified by triangulating off one or more signals emitted by a wearable device carried by the person.

3. The method of claim 1, wherein the location of the person in the room is identified by an in-room wave radar system that transmits one or more micro-wave or millimeter wave signals into the room and includes a plurality of distributed antenna for receiving a plurality of reflected signals reflected off the person in the room.

4. The method of claim 3, wherein the in-room wave radar system comprises a radar device, with the plurality of distributed antenna arranged along four sides of the radar device.

5. The method of claim 1, wherein the one or more conditions of the defined procedure further comprises hovering at the defined procedure location for at least a predetermined procedure time, and wherein the method further comprises determining whether the person hovers at the defined procedure location for at least the predetermined procedure time.

6. The method of claim 1, wherein the one or more conditions of the defined procedure further comprises making one or more predefined movements at the defined procedure location, and wherein the method further comprises determining whether the person makes the one or more predefined movements at the defined procedure location.

7. The method of claim 6, wherein the one or more predefined movements include a hand washing movement.

8. The method of claim 6, wherein the one or more predefined movements include a hand sanitizer pump movement.

9. The method of claim 1, wherein the defined procedure location corresponds to a sink in the room.

10. The method of claim 1, wherein the defined procedure location corresponds to a counter surface in the room.

11. The method of claim 1, further comprising:
detecting when the person enters the room, wherein the room includes a patient bed;
wherein the one or more conditions include, after entering the room, visiting the defined procedure location in the room before visiting the patient bed.

12. The method of claim 1, wherein the one or more conditions include repeatedly visiting the defined procedure location according to a predefined schedule.

13. The method of claim 1, further comprising providing an alert when one or more conditions of the defined procedure are not met.

14. A system for monitoring procedural compliance of staff in a facility, the system comprising:
a memory for storing a defined procedure that is to be performed by a person in a room of the facility, wherein the defined procedure includes one or more conditions, wherein the one or more conditions includes visiting a defined procedure location in the room;
a location identifier for identifying a location of a person in a room of the facility over time, wherein the location identifier is free from imaging pixels that together can be used to form a visually perceptible image of the room; and
a controller operatively coupled to the memory and the location identifier, the controller configured to determining whether the one or more conditions of the defined procedure have been met, including determining whether the person visited the defined procedure location in the room.

15. The system of claim 14, wherein the location identifier identifies the location of a person in a room by:
triangulating off one or more signals emitted by a device carried by the person; and/or
transmitting one or more signals into the room and receiving one or more corresponding reflected signals reflected off the person in the room.
